# EUROPEAN PATENT SPECIFICATION

(11) **EP 2 456 445 B1**
(45) Date of publication and mention of the grant of the patent: **14.05.2014**
(21) Application number: 11729376.1
(22) Date of filing: 24.06.2011
(51) Int. Cl.: A61K 31/695

(54) **AGENT FOR THE TREATMENT OF SKIN CONDITIONS**
MITTEL ZUR BEHANDLUNG VON HAUTLEIDEN
AGENT DE TRAITEMENT D'ÉTATS PATHOLOGIQUES CUTANÉS

(30) Priority: 25.06.2010 DE 102010025128
(43) Date of publication of application: 30.05.2012
(73) Proprietor: G. Pohl-Boskamp GmbH & Co. KG, 25551 Hohenlockstedt (DE)
(72) Inventor: BOSKAMP, Marianne, 25524 Bekmünde (DE)
(74) Representative: Maiwald Patentanwalts GmbH
(86) International application number: PCT/EP2011/003109
(87) International publication number: WO 2011/160844

(56) References cited:
- US-A- 2 298 273
- US-A1- 2007 031 348

## Description

### FIELD

The present invention relates to the use of at least one non-volatile silicone oil in combination with at least one spreading agent for the treatment of skin conditions which are typified by scales, plaques and scabs and are associated with punctiform bleedings occurring upon the removal of the scales, plaques and scabs, such as but not limited to cradle cap, seborrheic dermatitis, or psoriasis. The composition is useful to remove and facilitate removal of scales and consequently to reduce or prevent the incidence of punctiform bleeding, i.e., the formation of bloody tear points, in the affected areas of the skin.

### BACKGROUND

Various traditional methods are currently employed to treat cradle cap, seborrheic dermatitis, and psoriasis, although none to-date are satisfactory, especially with respect to punctiform bleeding. Some pediatricians recommend treating cradle cap with oils (usually overnight) such as mineral oils or plant-based oils (e.g., olive oil) which appear to be equivalent to mineral oils, followed by careful removal of any softened scales by hand with the aid of a soft brush or sponge and/or subsequent washing with a mild shampoo. Acidic components such as salicylic acid may be added to the oils for further softening the plaque in order to achieve detachment of the skin scales. An old home remedy recommends treatment with a bicarbonate paste applied to the affected skin areas for approximately 10 minutes, after which it is washed off. In some cases, the use of scale-loosening shampoos containing sulfur, selenium, zinc pyrithione, or salicylic acid is recommended. However, these shampoos may also cause additional skin irritation.

Some experts attribute cradle cap to an infection with *Malassezia* species. Yeasts are typically suspected to cause secondary infections in the case of cradle cap. Hence shampoos and creams containing ketoconazole are also recommended for the treatment of cradle cap. However, additional components contained in these shampoos may also cause skin irritation, so their use is recommended only in exceptional cases. The same applies for the use of ointments containing cortisone, which may be used for a short period of time in severe cases. Nevertheless, such medically active agents can have adverse side effects, especially in newborns and infants; thus these remedies are not desireable.

Various of these traditional remedies are also disclosed in the patent literature. For example, U.S. Patent Application No. 2004/0086477 describes a kit for the treatment of cradle cap. It includes multiple components and contains a composition for the pretreatment of the plaque which is a concentrated, highly viscous composition whose reduced fluidity is intended to prevent it from entering the child's eyes, ears, or mouth; a shampoo for removing any softened skin scales; and a moisturizing lotion for follow-up treatment of the affected skin areas. In addition, this kit also includes an implement for removing any loosened scales. Another remedy for cradle cap is disclosed in U.S. Patent No. 6,213,129 which describes a pump unit with a reservoir for the administration of baby oil and a comb attachment with which the softened skin scales may be removed. Unfortunately, these treatments are not able to completely remove the plaque in most cases, such that the treatment must be repeated multiple times. And even more unfortunate is the fact that these treatments do not minimize or eliminate punctiform bleeding as a side effect of scab or plaque removal.

Still other remedies, albeit ineffective, are available. For example, in addition to the administration of antibacterial or antifungal medications, the treatment of seborrheic dermatitis and psoriasis can also include the use of mineral or natural oils on the affected areas. These oils are applied either in an undiluted state or as emulsions. Moreover, dermatologists recommend topical treatments using shampoos, creams, or lotions containing antifungal, anti-inflammatory, sebosuppressive, or keratolytic ingredients such as, for example, ketoconazole, clotrimazole, lithium gluconate, terbinafine, or steroids. In addition, shampoos and lotions containing tar and vitamin D are available that are recommended for treatment of scaly skin conditions.

For example, a composition for the treatment of psoriasis containing tar as well as plant extracts as active ingredients is described in U.S. Patent No. 6,403,654. European Patent Application No. 1 957 167 discloses a spray containing vitamin D that is based on an oil matrix for the treatment of, among others, skin conditions such as psoriasis or seborrheic dermatitis. International Patent Application No. WO 01/17526 discloses a method for the treatment of skin conditions such as psoriasis, seborrheic dermatitis, and cradle cap including the administration of a topical preparation essentially containing 5 to 100 wt.% of glyceryl fatty acid esters (e.g., triacetin) and an inert carrier substance. The fatty acid ester serves to lower the pH value, which is typically basic for scaly skin areas, and purportedly supports the healing process in the affected skin areas by stabilizing slightly acidic pH values.

Once again, however, the treatment methods mentioned above frequently show only short-term success, for which reason treatment must be regularly repeated. In addition, some of the oils applied in these treatments are very viscous, such that it is not easy to effectively distribute the oils over the affected skin areas, nor is it easy to remove them later. Particularly in psoriasis patients, the removal of scales using such treatment methods regularly leads to discrete bleeding spots known as Auspitz's sign and the risk of infection associated therewith is thus heightened. In addition, these compositions contain some active ingredients that are particularly inappropriate for use on small children.

Therefore, in the treatment of skin conditions typified by scales, plaques and scabs and associated with punctiform bleeding, such as but not limited to cradle cap, seborrheic dermatitis, and psoriasis, the need continues to exist for effective and gentle treatment methods using compositions that are not only easy to apply but do not contain any pharmaceutically or medically active ingredients that penetrate the skin, all the while diminishing or preventing punctiform bleeding.

### SUMMARY

Provided herein are elegant, safe and effective compositions and methods for treating and alleviating skin conditions typified by scales, plaques and scabs and associated with punctiform bleeding, such as but not limited to cradle cap, seborrheic dermatitis and psoriasis. These skin conditions are typified by scales, plaques and scabs and are associated with punctiform bleeding and/or redness of the skin during or after the removal of the scales, plaque or scabs associated with these conditions.

Accordingly, in various embodiments, compositions and methods are provided for treating a scaly skin condition. The compositions can include at least one non-volatile silicone oil in combination with at least one spreading agent. The incidence of punctiform bleeding, redness of the skin, and formation of bloody tear points on the skin during or after removal of the scales is diminished or prevented when using these compositions.

Embodiments of the invention can include one or more of the following features, by way of non-limiting example:
The composition *per se* can be a solution having a viscosity of 5 to 25 cSt, such as, for example, 10 to 15 cSt.

The composition can include at least one non-volatile silicone oil selected from poly dialkyl siloxanes, poly diaryl siloxanes, and poly alkylaryl siloxanes. For example, the non-volatile silicone oil can be dimethicone with a viscosity of 90 cSt to 200 cSt, such as, for example, dimethicone with a viscosity of 90 cSt to 120 cSt.

The spreading agent can be selected from oleyl oleate, ethylhexyl stearate, hexyl laurate, dibutyl adipate, dicaprylyl carbonate, ethylhexyl palmitate, oleyl erucate, coco-caprylate/caprate, dicaprylyl ether, propylheptyl caprylate, cetearyl isononanoate, decyl oleate, and silicone oils with a viscosity of < 12 cSt. For example, the spreading agent can be dicaprylyl carbonate and/or dimethicone with a viscosity between 0.5 and 12 cSt.

The composition can be in the form of a solution containing 20 to 80 wt.% dicaprylyl carbonate, 20 to 80 wt.% dimethicone with a viscosity of 100 cSt, and 1 to 60 wt.% dimethicone with a viscosity of 10 cSt, with all percentages by weight relative to the total composition. For example, the composition can be in the form of a solution containing 60 wt.-% dicaprylyl carbonate, 20 wt.% dimethicone with a viscosity of 100 cSt, and 20 wt.% dimethicone with a viscosity of 10 cSt, with all percenttages by weight relative to the total composition. The composition also can be in the form of a solution containing 50 wt.% dicaprylyl carbonate, 40 wt.-% dimethicone with a viscosity of 100 cSt, and 10 wt.% dimethicone with a viscosity of 10 cSt, with all percentages by weight relative to the total composition.

The method can include the step of contacting an area of affected skin with the composition to diminish or prevent punctiform bleeding or redness associated with scale, plaque, or scab removal from the affected skin.

The method can include contacting the skin with the composition; allowing the scales, plaques or scabs on the affected skin to sufficiently soften; and removing the scales, plaques or scabs with little or no punctiform bleeding or redness.

The method can include reapplying the composition as necessary, for example, reapplying the composition once daily for 2-3 days.

### DETAILED DESCRIPTION

Provided herein are elegant, safe and effective compositions and methods for treating and alleviating skin conditions typified by scales, plaques and scabs and are associated with punctiform bleeding, such as but not limited to cradle cap, seborrheic dermatitis and psoriasis. Surprisingly, it was found that the topical application of at least one non-volatile silicone oil combined with a suitable spreading agent supports the alleviation and treatment of cradle cap and other scaly skin conditions (e.g., seborrheic dermatitis and psoriasis) and relieves the discomfort and risk of infection associate with such conditions. Most interestingly the present invention does not only facilitate the removal of the skin or sebum scales typical for the manifestation of the condition itself but also prevented the occurrence of bloody tear spots on the skin, which is a common and unfortunate side effect of prior art treatments. The extraordinary efficacy of the stated combination of at least one non-volatile silicone oil and at least one spreading agent is further unexpected because these results are accomplished using pharmaceutically and medically inert ingredients, i.e., without using any pharmacologically active ingredients. Heretofore, it was believed that inert ingredients should be accompanied by a medically or pharmaceutically active ingredient in order for a composition to be effective. Thus the present invention is unexpected and surprising in at least this one feature.

The present invention permits the gentle removal of scales or scabs without the formation of bloody tear points in the affected skin areas and prevention of punctiform bleeding as an adverse treatment outcome; and, also decreases the risk of secondary infection and facilitates subsequent treatment with preparations containing active ingredients, if necessary. These preparations containing active ingredients may be distributed more easily over the skin and penetrate the skin better after a pretreatment with the composition of the present invention, thus allowing the use of smaller amounts of active ingredient. The latter is particularly advantageous for the treatment of psoriasis, in which other preparations are repeatedly used that have serious side effects.

Cradle cap is the colloquial term for a crusty skin condition on the face and scalp during infancy. It manifests as greasy and oily plaque, scabs or scales. Some experts assign cradle cap to atopic dermatitis, some to seborrheic dermatitis. While cradle cap which is associated with atopic dermatitis/eczema causes itching conditions and is rarely seen in infants before the third month of life, infantile seborrheic dermatitis can appear within the first weeks after birth. The cause for cradle cap is controver-sially discussed within science and is not yet fully understood. These skin conditions can disappear on their own without treatment after a period of weeks or months. In some cases, however, the condition does not heal completely until the child is of elementary school age. Although the condition is usually not harmful *per se,* due to the unsightly appearance of the scales, scabs and plaque, the unpleasant smell, the associated itching, and the ever-present risk of infection, there is a great interest in treating the condition in order to eliminate the symptoms or at least reduce them to a minimum.

Seborrheic dermatitis during adulthood and psoriasis are also characterized by the pronounced formation of scales on affected areas of skin. The causes of these conditions have not yet been fully discovered. However, it is assumed that a genetic predisposition is present in affected patients. While seborrheic dermatitis is associated with elevated activity of the sebaceous glands, psoriasis is characterized by an increased proliferation of skin cells. In both cases, the skin exhibits a scaly appearance.

The skin appearance of patients suffering from cradle cap, seborrheic dermatitis or psoriasis is often very similar. Cradle cap and seborrheic dermatitis can cause occasional pruritus, dandruff, and yellow, greasy scaling along the hairline and on the face. Psoriasis is classified in different types - plaque, guttate, inverse, pustular and erythrodermic - with the most common form being plaque psoriasis. Plaque psoriasis typically appears as raised areas of inflamed skin covered with silvery white scaly skin.

A major symptom of cradle cap, seborrheic dermatitis and psoriasis is the formation of plaques and scales. In some cases, cradle cap and seborrheic dermatitis are difficult to distinguish from psoriasis in the scalp area. In adult patients with a diathesis for psoriasis, seborrheic dermatitis may even precede or be associated with psoriasis (called sebopsoriasis). A typical sign of psoriasis is the appearance of pinpoint bleeding when the plaques are scraped off, the so called Auspitz's sign. But bloody tear points can also occur upon removal of scales of patients with cradle cap or seborrheic dermatitis.

"Bloody tear points" result when, after the removal of the surface skin scales, superficial blood vessels are damaged while removing the last layer of scales over the epidermis. This damage is visible as punctiform areas of bleeding on the surface of the skin. In the present description, the terms "bloody tear point" and "punctiform bleeding" are used synonymously. Punctiform bleeding are associated with the diseases for which the present invention is most efficacious, namely the diseases described earlier of cradle cap, seborrheic dermatitis and psoriasis. The skilled practitioner will undoubtedly appreciate that other skin diseases typified by scales, plaques and scabs and associated with punctiform bleeding are also suitable for treatment with the compositions and methods of the present invention. In addition to punctiform bleeding, removal of surface skin scales, plaque or scabs can cause other associated and undesired side effects, such as varying degrees of redness and irritation as described elsewhere herein.

As used herein, the term "low viscosity" refers to a liquid or a solution having a viscosity below about 50 cSt. Low viscosity, oily liquids and solutions have a low surface tension and therefore have good creep properties, such as, for example penetration into microscopic crevasses in the affected areas of the skin.

As used herein, the term "medium viscosity" refers to a liquid or a solution having a viscosity of about 50 cSt to about 500 cSt. Medium viscosity oily liquids and solutions spread evenly over the skin and serve to lubricate and soften the skin.

As used herein, the term "non-volatile" refers to a liquid or solution that does not readily evaporate. As a result, non-volatile liquids and solutions form a protective barrier when applied the skin. Non-volatile liquids or solutions have a boiling point above 200 °C. For example, in the present invention, the non-volatile silicone oil is preferably a medium-viscosity dimethicone with a viscosity of 90 to 200 cSt; the use of a dimethicone having a viscosity between 90 and 120 cSt is particularly preferred.

The composition according to the present invention contains at least one non-volatile silicone oil as an active component along with at least one suitable pharmaceutically compatible and toxicologically harmless spreading agent. The composition can optionally contain one or more pharmaceutically compatible excipients. The composition of the present invention contains substantially no water and is thus not an emulsion. Heretofore, it was believed that a preferred composition was an emulsion and that such properties were necessary for success, so the present invention is unexpected and surprising in yet another feature.

In the present invention, the non-volatile silicone oil is preferably a medium-viscosity dimethicone with a viscosity of 90 to 200 cSt; the use of a dimethicone having a viscosity between 90 and 120 cSt is particularly preferred.

In the present invention, the spreading agent supports the distribution of the dimethicone on the skin as well as its penetration into the scaly layer, thus facilitating the application of the composition. Particularly preferred spreading agents include low-viscosity dimethicones having a viscosity of < 12 cSt, with dimethicone having a viscosity of 1 cSt, dimethicone having a viscosity of 10 cSt and dicaprylyl carbonate being particularly preferred. Thus in accordance with the present invention, a preferred composition contains 20 to 80 wt.% of at least one low-viscosity silicone oil having a viscosity less than 12 cSt as a spreading agent, as well as at least one low-viscosity ester, at least one low-viscosity ether, or mixtures thereof. Suitable low-viscosity esters are oleyl oleate, ethylhexyl stearate, hexyl laurate, dibutyl adipate, dicaprylyl carbonate, ethylhexyl palmitate, oleyl erucate, coco caprylate/caprate, propylheptyl caprylate, cetearyl isononanoate, and decyl oleate, as well as mixtures thereof. In certain embodiments, dicaprylyl ether or mixtures thereof with the aforementioned esters and/or dimethicones can be used as spreading agents.

In various embodiments, the use of the dicaprylyl carbonate marketed under the name Cetiol^{®} CC (Cognis), dimethicone with a viscosity of 1 cSt (Belsil^{®} 1 cst), or dimethicone with a viscosity of 10 cSt (Xiameter^{®} 200PMX), and mixtures thereof as a spreading agent is particularly preferred.

The compositions *per se* according to the present invention are advantageously characterized by a low viscosity between 5 and 25 cSt, preferably between 10 and 20 cSt, with the viscosity of the compositions *per se* between 10 and 15 cSt being particularly preferred.

Compositions that are particularly suitable within the meaning of the present invention are solutions that can be dispensed in the form of drops or as a spray, either an aerosol spray or a pump spray. They guarantee an even topical application of a homogeneous film of the active ingredient on the affected skin areas in a comfortable fashion. The compositions according to the present invention are applied to the affected skin areas.

It has been shown that a single administration, optionally two to three administrations, of dimethicone in a composition according to the present invention removes the scales of cradle cap in a residue-free fashion without any follow-up treatment or by simply rinsing with warm water, i.e., no new cradle cap occurred within the observation period. Moreover, removal of cradle cap is achieved with little or no punctiform bleeding or redness of the skin. Importantly, this was accomplished with pharmaceutically inert ingredients thereby reducing exposure of the patient to undue chemicals or ingredients with potentially adverse side effects. Here, the composition was administered in at least a sufficient amount to fully wet the damaged area of the skin.

Relief of symptoms was also observed when the composition according to the present invention was used on scaly skin areas associated with seborrheic dermatitis or psoriasis. In these cases, however, it was necessary to repeat the treatment after approximately one week. It should be particularly emphasized that no bloody tear points were observed in the removal of scales when treating psoriasis patients. Again, it is therefore possible to reduce the risk of secondary infection by treatment with an inert ingredient such as a non-volatile silicone oil. This is also advantageous when the further treatment of the skin condition requires the application of preparations containing active ingredients, which may thus be more easily applied and, optionally, may be used in smaller amounts, which advantageously supports treatment and lessens any side effects that may occur.

Compositions containing silicone oil that are suitable within the meaning of the present invention are present as a solution and contain 10 to 90 wt.% dimethicone with a viscosity of 90 to 200 cSt as well as 10 to 90 wt.% of a spreading agent. Most preferably, the compositions of the present invention contain 10 to 90 wt.-% dimethicone with a viscosity of 90 to 120 cSt along with 1 to 60 wt.-% dimethicone with a viscosity of < 12 cSt as well as 20 to 80 wt.% dicaprylyl carbonate. Compositions containing 20 wt.-% dimethicone with a viscosity of 100 cSt, 20 wt.-% dimethicone with a viscosity of 10 cSt, and 60 wt.% dicaprylyl carbonate, but also compositions containing 40 wt.% dimethicone with a viscosity of 100 cSt, 10 wt.% dimethicone with a viscosity of 10 cSt, and 50 wt.-% dicaprylyl carbonate are also preferred.

Unless stated otherwise, the percentages by weight are based on the weight of the total composition. In this description, the viscosity figures are based on values measured at 25°C. The viscosity values are understood to be the stated values ± 10%.

The present invention is described in greater detail with reference to the following examples.

### EXAMPLES

### Example 1

| Ingredient | Amount in g |
|---|---|
| Dimethicone 100 cSt | 40.00 |
| Dimethicone 10 cSt | 10.00 |
| Dicaprylyl carbonate* | 50.00 |
| Total of individual substances | 100.00 |

| | |
|---|---|
| * Commercially available as Cetiol^{®} CC | |

### Example 2

| Ingredient | Amount in g |
|---|---|
| Dimethicone 100 cSt | 20.00 |
| Dimethicone 10 cSt | 20.00 |
| Dicaprylyl carbonate* | 60.00 |
| Total of individual substances | 100.00 |

| | |
|---|---|
| * Commercially available as Cetiol^{®} CC | |

The solutions are produced by mixing the components thoroughly with one another using routine formulary methodologies.

### Example 3

A five-month-old boy suffering from a severe case of cradle cap was treated using the solution according to Example 2. The boy was a normally developing baby whose cradle cap had been treated unsuccessfully with olive oil over the course of several weeks. The affected areas were treated twice with the solution according to Example 2 at an interval of two days. For the treatments, the scabbed areas of skin were sprinkled with two to three milliliters of the solution. The solution penetrated quickly and completely into the scaly layer and was fully absorbed thereby. The oil was not subsequently washed out. Loose flakes were simply removed by hand. After the second treatment, the scales had completely detached. No punctiform bleedings occurred during and after the treatment. The cradle cap did not return.

### Example 4

In a 41-year-old male patient a scaly plaque , behind the ear which was attributed to sebopsoriasis was treated with a spray composition containing the solution according to Example 1. A visible reduction in skin scales was observed. No punctiform bleedings occurred.

The examples given above show that the compositions containing various preferred amounts of low and medium viscosity dimethicone according to the present inventtion are able to remove cradle cap gently without leaving residue and with no incidences of punctiform bleeding. In the treatment of psoriasis and other scaly skin conditions, treatment with the compositions containing dimethicone according to the present invention caused relief of symptoms: It was possible to remove scales easily from the skin without the formation of punctiform areas of bleeding and without risk of infection resulting therefrom.

### Example 5

A clinical investigation of the solution according to Example 2 will be performed in about 20 male or female infants/children, ages 3 to 36 months, with cradle cap on the scalp.

The target area for treatment is the worst affected area on the scalp. The target area will receive at least one treatment and up to three treatments during the investigation period. Adjoining areas will also be treated, but only the target area will be assessed. The treatment will be performed by the parent(s)/legal guardian(s) according to prepared instructions under an investigator's knowledgeable supervision.

All subjects will receive at least one treatment on day 1. In case of no immediate treatment success, further treatments might be necessary on days 2 and 3. The composition will be a solution according to Example 2 for topical application once or up to three times over a maximum investigation period of 3 consecutive days per individual.

Treatment dosages will vary depending on the size of the affected area on the scalp. Generally, enough solution will be applied to cover the affected area.

Clinical assessment will be performed using a 5-point scale from 0 to 3 (0, 0.5, 1,2 and 3). Treatment success is defined as reduction of the scaling baseline score of at least two grades (meaning a resulting grade of 0.5 or 0 when the initial score was 2, or a resulting grade of 1 or 0.5 or 0 when the initial score was 3). Treatment of the affected area will be continued until treatment success is achieved, or up to a maximum of three treatments. See Tables 1 and 2 for further explanation and clinical details.

Clinical assessment and photo documentation of the target area will be performed on days 1, 2 and 8 (± day). In case treatment success does not occur by day 2 or 3, clinical assessment and photo documentation will also be performed on days 3 and 4, respectively.

Storage temperature of the solution is not to exceed 25 °C.

On day 1 and in case of no treatment success also on days 2 and 3 the solution will be applied to the affected areas on the scalp as described in the prepared instructions. Briefly, the solution will be applied dropwise on a dry scalp using a pipette. The pipette should not contact the scalp. Excessive solution will be swabbed with a soft paper tissue. The solution will be spread by gentle massage until the area is completely moistened. The solution should be left on the scalp for at least 3 hours or can be left on over night.

After at least 3 hours, and before the next visit at the clinical site (day 2, 3 and 4), the solution should be washed out with a standard shampoo, which will be provided by the sponsor and handed out to the parent(s)/legal guardian(s).

On the first day of the clinical investigation, the target area will be selected and the location on the scalp documented. The outline of the affected area and the target area will be traced onto a transparent plastic sheet. Baseline scores for scaling will be given in the target area of the scalp and baseline scores for secondary parameters will also be assessed. Application of the investigational composition will be performed by the parent(s)/legal guardian(s). The parent(s)/legal guardian(s) will then wash off the investigational composition at home, at the earliest 3 hours after application.

On day 2, subjects will return for clinical assessment of scaling and secondary parameters. Only in case that there is no treatment success on day 2, treatment will be repeated (as performed on day 1) and subjects will return for clinical assessment of scaling and secondary parameters on day 3. In case that there is still no treatment success on day 3, treatment will be repeated again and subjects will return for assessment of scaling and secondary parameters on day 4.

On day 8 (± 1), subjects will return for a follow-up visit. Scaling and secondary parameters will be scored for assessment of persistence of treatment effect and safety. Photos will also be taken during that follow-up visit.

The target area of the scalp will be scored for scaling and as secondary parameter bloody tear points and redness on day 1, 2 and 8 (± 1), and if applicable on day 3 and 4 according to the following scale:

**Table 1: Scaling**

| **Score** | **Scaling** |
|---|---|
| 0 (none) | no plaques, no flakes in the target area |
| 0.5 (very mild) | target area shows only a few single soft plaques |
| 1 (mild) | target area shows single spots or full soft plaque, soft loosened flakes |
| 2 (moderate) | target area shows large single spots or a full plaque; some loosened flakes possible |
| 3 (severe) | target area shows a full thick, tight plaques, no loosened flakes |

**Table 2: Redness**

| **Score** | **Redness** |
|---|---|
| 0 (none) | no signs of redness |
| 0.5 (very mild) | very slight redness |
| 1 (mild) | slight redness |
| 2 (moderate) | clear redness |
| 3 (severe) | intensive redness |

Scaling as described in Table land redness as described in Table 2 will be among the clinical endpoints measured and will be assessed separately. The scoring will be performed by two trained investigators to assure comparable grading. The presence or absence of bloody tear points will also be documented.

Photo documentation of the scalp will be performed before application of the investigational composition using a digital camera on day 1 and on day 2 and 8 (±1) and in case of repeated treatment(s) on day 3 and 4, respectively. The photographs will be taken under standardized conditions (e.g., distance, illumination).

It is expected that treatment of patients with the compositions and methods of the present invention will result in a significantly diminished scaling as well as a diminished incidence of punctiform bleeding and redness.

The aspects, embodiments, features, and examples of the invention are to be considered illustrative in all respects and are not intended to limit the invention, the scope of which is defined only by the claims. Other embodiments, modifications, and usages will be apparent to those skilled in the art without departing from the spirit and scope of the claimed invention.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components and can be selected from a group consisting of two or more of the recited elements or components.

The use of the terms "include," "includes," "including," "have," "has," or "having" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise. In addition, where the use of the term "about" is before a quantitative value, the present teachings also include the specific quantitative value itself, unless specifically stated otherwise. As used herein, the term "about" refers to a ±10% variation from the nominal value, unless otherwise stated.

It should be understood that the order of steps or order for performing certain actions is immaterial so long as the present teachings remain operable. Moreover, two or more steps or actions may be conducted simultaneously.

Where a range or list of values is provided, each intervening integer and, where appropriate, decimal between the endpoints of that range or list of values is individually contemplated and is encompassed within the invention as if each value were specifically enumerated herein. In addition, smaller ranges between and including the endpoints of a given range are contemplated and encompassed within the invention. The listing of exemplary values or ranges is not a disclaimer of other values or ranges between and including the endpoints of a given range.

## Claims

1. A composition comprising at least one non-volatile silicone oil in combination with at least one spreading agent for use in the treatment of skin conditions in mammals typified by scales, plaques and scabs and associated with punctiform bleeding during the removal of scales, plaque or scabs, wherein the incidence of punctiform bleeding and formation of bloody tear points on the skin during removal of the scales is diminished or prevented.

2. The composition as recited in Claims 1, **wherein** it is present in the form of a solution with a viscosity of 5 to 25 cSt.

3. The composition as recited in Claim 2, **wherein** the composition has a viscosity of 10 to 15 cSt.

4. The composition as recited in one of Claims 1 to 3, **wherein** the at least one non-volatile silicone oil is selected from the group containing poly dialkyl siloxanes, poly diaryl siloxanes, and poly alkylaryl siloxanes.

5. The composition as recited in one of Claims 1 to 4, **wherein** the non-volatile silicone oil is dimethicone with a viscosity of 90 cSt to 200 cSt.

6. The composition as recited in Claim 5, **wherein** the silicone oil is dimethicone with a viscosity of 90 cSt to 120 cSt.

7. The composition as recited in one of Claims 1 to 6, **wherein** the spreading agent is selected from the group including oleyl oleate, ethylhexyl stearate, hexyl laurate, dibutyl adipate, dicaprylyl carbonate, ethylhexyl palmitate, oleyl erucate, coco-caprylate/caprate, dicaprylyl ether, propylheptyl caprylate, cetearyl isononanoate, decyl oleate, and silicone oils with a viscosity of < 12 cSt.

8. The composition as recited in Claim 7, **wherein** dicaprylyl carbonate and/or dimethicone with a viscosity between 0.5 and 12 cSt is included as the spreading agent.

9. The composition as recited in one of Claims 1 to 8, **wherein** it is present in the form of a solution containing 20 to 80 wt.% dicaprylyl carbonate, 20 to 80 wt.-% dimethicone with a viscosity of 100 cSt, and I to 60 wt.% dimethicone with a viscosity of 10 cSt, with all percentages by weight relative to the total composition.

10. The composition as recited in one of Claims 1 to 9, **wherein** the composition contains 60 wt.-% dicaprylyl carbonate, 20 wt.-% dimethicone with a viscosity of 100 cSt as well as 20 wt.% dimethicone with a viscosity of 10 cSt, with all percentages by weight relative to the total composition.

11. The composition of claim 1 wherein the skin condition is selected from the group consisting of: seborrheic dermatitis, psoriasis, and cradle cap.

## Patentansprüche

1. Zubereitung umfassend wenigstens ein nicht-flüchtiges Silikonöl in Kombination mit wenigstens einem Spreitmittel zur Verwendung in der Behandlung von Hautzuständen bei Säugetieren vorliegend als Schuppen, Beläge und Schorf sowie verbunden mit punktförmigen Blutungen während der Entfernung der Schuppen, Beläge oder des Schorfs, **wobei** das Auftreten der punktförmigen Blutungen und die Bildung von blutigen Abrisspunkten auf der Haut während des Entfernens der Schuppen reduziert oder verhindert wird.

2. Zubereitung gemäß Anspruch 1, vorliegend in Form einer Lösung mit einer Viskosität von 5 bis 25 cSt.

3. Zubereitung gemäß Anspruch 2, **wobei** die Viskosität der Zubereitung 10 bis 15 cSt beträgt.

4. Zubereitung gemäß einem der Ansprüche 1 bis 3, **wobei** das wenigstens eine nicht-flüchtige Silikonöl ausgewählt ist aus der Gruppe umfassend Polydialkylsiloxane, Polydiarylsiloxane und Polyalkylarylsiloxane.

5. Zubereitung gemäß einem der Ansprüche 1 bis 4, **wobei** das nicht-flüchtige Silikonöl Dimethicon einer Viskosität von 90 bis 200 cSt ist.

6. Zubereitung gemäß Anspruch 5, **wobei** das Silikonöl Dimethicon einer Viskosität von 90 bis 120 cSt ist.

7. Zubereitung entsprechend einem der Ansprüche 1 bis 6, **wobei** das Spreitmittel ausgewählt ist aus der Gruppe umfassend Oleyloleat, Ethylhexylstearat, Hexyllaurat, Dibutyladipat, Dicaprylylcarbonat, Ethylhexylpalmitat, Oleylerucat, Coco-Caprylat/Caprat, Dicaprylylether, Propylheptylcaprylat, Cetearylisononanoat, Decyloleat und Silikonöle mit einer Viskosität von < 12 cSt.

8. Zubereitung gemäß Anspruch 7, **wobei** Dicaprylylcarbonat und/oder Dimethicon mit einer Viskosität zwischen 0,5 und 12 cSt als Spreitmittel enthalten ist.

9. Zubereitung gemäß einem der Ansprüche 1 bis 8, **wobei** diese in Form einer Lösung vorliegt umfassend 20 bis 80 Gew.-% Dicaprylylcarbonat, 20 bis 80 Gew.-% Dimethicon einer Viskosität von 100 cSt und 1-60 Gew.-%, jeweils bezogen auf die Gesamtzubereitung, Dimethicon einer Viskosität von 10 cSt enthält.

10. Zubereitung gemäß einem der Ansprüche 1 bis 9, **wobei** die Zubereitung 60 Gew.-% Dicaprylcarbonat, 20 Gew.-% Dimethicon einer Viskosität von 100 cSt sowie 20 Gew.-%, jeweils bezogen auf die Gesamtzubereitung, Dimethicon einer Viskosität von 10 cSt enthält.

11. Zubereitung gemäß Anspruch 1, **wobei** die Hautzustände ausgewählt sind aus der Gruppe bestehend aus seborrhoischer Dermatitis, Psoriasis und Milchschorf.

## Revendications

1. Composition contenant au moins une huile de silicone non volatile en combinaison avec au moins un agent d'enduction à utiliser dans le traitement d'états pathologiques cutanés chez des mammifères **caractérisés par** des écailles, des plaques et des croûtes et associés à un saignement punctiforme pendant l'enlèvement des écailles, des plaques ou des croûtes, dans lequel l'incidence du saignement punctiforme et la formation de points de déchirure saignants sur la peau pendant l'enlèvement des écailles sont diminuées ou empêchées.

2. Composition selon la revendication 1, dans laquelle elle est présente sous la forme d'une solution présentant une viscosité comprise entre 5 cSt et 25 cSt.

3. Composition selon la revendication 2, dans laquelle la composition présente une viscosité comprise entre 10 cSt et 15 cSt.

4. Composition selon l'une quelconque des revendications 1 à 3, dans laquelle ladite au moins une huile de silicone non volatile est sélectionnée dans le groupe comprenant des poly-dialkyle siloxanes, des poly-diaryle siloxanes et des poly-alkylaryle siloxanes.

5. Composition selon l'une quelconque des revendications 1 à 4, dans laquelle l'huile de silicone non volatile est le diméthicone présentant une viscosité comprise entre 90 cSt et 200 cSt.

6. Composition selon la revendication 5, dans laquelle l'huile de silicone est le diméthicone présentant une viscosité comprise entre 90 cSt et 120 cSt.

7. Composition selon l'une quelconque des revendications 1 à 6, dans laquelle l'agent d'enduction est sélectionné dans le groupe comprenant l'oléate d'oléyle, le stéarate d'éthylhexyle, le laurate d'hexyle, l'adipate de dibutyle, le carbonate de dicaprylyle, le palmitate d'éthylhexyle, l'érucate d'oléyle, le coco-caprylate/caprate, l'éther de dicaprylyle, le caprylate de propylheptyle, l'isononanoate de cétéaryle, l'oléate de décyle, ainsi que des huiles de silicone présentant une viscosité inférieure à 12 cSt.

8. Composition selon la revendication 7, dans laquelle le carbonate de dicaprylyle et/ou le diméthicone présentant une viscosité comprise entre 0,5 cSt et 12 cSt est/sont inclus comme agent(s) d'enduction.

9. Composition selon l'une quelconque des revendications 1 à 8, dans laquelle elle est présente sous la forme d'une solution contenant de 20 % en poids à 80 % en poids de carbonate de dicaprylyle, de 20 % en poids à 80 % en poids de diméthicone présentant une viscosité de 100 cSt, et de 1 % en poids à 60 % en poids de diméthicone présentant une viscosité de 10 cSt, tous les pourcentages étant des pourcentages en poids par rapport à la composition totale.

10. Composition selon l'une quelconque des revendications 1 à 9, dans laquelle la composition contient 60 % en poids de carbonate de dicaprylyle, 20 % en poids de diméthicone présentant une viscosité de 100 cSt, ainsi que 20 % en poids de diméthicone présentant une viscosité de 10 cSt, tous les pourcentages étant des pourcentages en poids par rapport à la composition totale.

11. Composition selon la revendication 1, dans laquelle l'état pathologique cutané est sélectionné dans le groupe comprenant la dermatite séborrhéique, le psoriasis et le casque séborrhéique.
